# EUROPÄISCHE PATENTSCHRIFT

(11) **EP 0 905 379 B1**
(45) Veröffentlichungstag und Bekanntmachung des Hinweises auf die Patenterteilung: **14.05.2003**
(21) Anmeldenummer: 97810706.8
(22) Anmeldetag: 25.09.1997
(51) Int. Cl.: F04D 1/00, F04D 29/04, F04D 13/14, F04D 1/06, F04D 13/06, A61M 1/10

(54) **Zentrifugalpumpe und Zentrifugalpumpenanordnung**
Centrifugal pump and centrifugal pump arrangement
Pompe centrifuge et système des pompes centrifuges

(43) Veröffentlichungstag der Anmeldung: 31.03.1999
(73) Patentinhaber: Levitronix LLC, Waltham, MA 02451 (US)
(72) Erfinder: Schöb, Reto, Dr., 8604 Volketswil (CH)
(74) Vertreter: Sulzer Management AG

(56) Entgegenhaltungen:
- EP-A- 0 447 106
- WO-A-94/13955
- WO-A-96/31934
- DE-C- 924 188
- US-A- 2 700 343
- US-A- 3 272 129
- US-A- 4 526 507
- US-A- 5 055 005
- US-A- 5 399 074
- GUPTA: "Double Inlet Pump" IBM TECHNICAL DISCLOSURE BULLETIN, Bd. 13, Nr. 12, Mai 1971, Seite 3710 XP002058163

## Beschreibung

Die Erfindung betrifft eine Zentrifugalpumpe zum Fördern eines Fluids gemäss dem Oberbegriff des unabhängigen Anspruchs 1 sowie eine Zentrifugalpumpenanordnung. Insbesondere betrifft die Erfindung eine Zentrifugalpumpe und eine Zentrifugalpumpenanordnung zum Fördern von Blut.

In der WO-A-96/31934 wird eine Rotationspumpe und insbesondere eine Zentrifugalpumpe offenbart, bei der das Flügelrad im Innern des Pumpengehäuses durch magnetische Kräfte schwebend gelagert ist und durch ein Drehfeld angetrieben wird, das von einem ausserhalb des Pumpengehäuses angeordneten Stator erzeugt wird. Dabei ist nach dem Prinzip des sogenannten lagerlosen Motors der Stator als Lager-und Antriebsstator ausgebildet und der im Flügelrad integrierte Rotor als Lager- und Antriebsrotor. Solche Pumpen sind insbesondere für solche Anwendungen vorteilhaft, bei denen das zu fördernde Fluid nicht verunreinigt werden darf, beispielsweise zum Fördern biologischer Flüssigkeiten wie Blut oder hochreiner Flüssigkeiten wie Reinstwasser. Zudem eignen sich solche Rotationspumpen zum Fördern aggressiver Flüssigkeiten, die mechanische Lager in kurzer Zeit zerstören würden.

Derartige Zentrifugalpumpen werden beispielsweise als Blutpumpen zur Aufrechterhaltung des Blutkreislaufs während Operationen am offenen Herzen eingesetzt. Auch ist es möglich, solche Pumpen als künstliches Herz einzusetzen. Dabei ist es notwendig, mindestens zwei separate Pumpen einzusetzen. Die eine Pumpe übernimmt die Funktion des linken Ventrikels und erhält den Körperkreislauf aufrecht, während die andere Pumpe die Funktion des rechten Ventrikels übernimmt und den Lungenkreislauf aufrechterhält. Die Verwendung mindestens zweier separater Pumpen zur Ausübung der Herzfunktion bringt jedoch einen relativ grossen Platzbedarf mit sich, der sich insbesondere im Hinblick auf eine Implantation solcher Pumpen als künstliches Herz als einschränkend erweist.

Es ist daher eine Aufgabe der vorliegenden Erfindung, eine Zentrifugalpumpe bereitzustellen, die das Fluid in zwei unterschiedlichen Fluidsystemen fördern kann. Die Zentrifugalpumpe soll dabei sehr kompakt ausgestaltet sein und eine hohe Förderleistung ermöglichen. Ferner soll sie insbesondere eine Ausgestaltung als Blutpumpe ermöglichen, welche die Funktion des Herzens übernehmen kann. Eine weitere Aufgabe der Erfindung ist es, eine Pumpenanordnung bereitzustellen, die ein hohes Mass an Sicherheit im Hinblick auf Betriebsstörungen aufweist.

Diese Aufgaben werden gelöst durch eine Zentrifugalpumpe, die durch die Merkmale des unabhängigen Anspruchs 1 gekennzeichnet ist bzw. durch eine Zentrifugalpumpenanordnung, die durch die Merkmale des unabhängigen Anspruchs 13 gekennzeichnet ist.

Durch die erfindungsgemässe Anordnung der Auslässe, nämlich bezüglich der axialen Richtung versetzt zueinander, kann die erfindungsgemässe Zentrifugalpumpe die Funktion zweier Pumpen übernehmen. Sie fördert zum einen mittels des ersten Satzes von Flügeln das Fluid von dem ersten Einlass zum ersten Auslass und zum anderen mittels des zweiten Satzes von Flügeln das Fluid vom zweiten Einlass zum zweiten Auslass. Somit kann die erfindungsgemässe Zentrifugalpumpe das Fluid in zwei unterschiedlichen Fluidsystemen fördern.

Die Zentrifugalpumpenanordnung mit mindestens zwei Zentrifugalpumpen ermöglicht ein hohes Mass an Sicherheit. Jede der beiden Zentrifugalpumpen alleine kann das Fluid in den beiden Fluidsystemen fördern, sodass selbst beim Ausfall einer der beiden Zentrifugalpumpen die andere Zentrifugalpumpe die Funktionstüchtigkeit der Zentrifugalpumpenanordnung gewährleistet. Diese Redundanz ist insbesondere auch im Hinblick auf medizinische Anwendungen besonders vorteilhaft.

Weitere vorteilhafte Massnahmen und Ausgestaltungen ergeben sich aus den abhängigen Ansprüchen.

Im folgenden wird die Erfindung anhand von Ausführungsbeispielen und anhand der Zeichnung näher erläutert. In der schematischen nicht massstäblichen Zeichnung, in der identische oder von der Funktion her gleichwertige Teile mit den gleichen Bezugszeichen versehen sind, zeigen:
- Fig. 1:: einen Längsschnitt durch ein erstes Ausführungsbeispiel der erfindungsgemässen Zentrifugalpumpe,
- Fig. 2:: einen Querschnitt durch das erste Ausführungsbeispiel entlang der Schnittlinien II-II bzw. IIa-IIa in Fig. 1,
- Fig. 3:: einen Querschnitt durch das erste Ausführungsbeispiel entlang der Schnittlinien III-III bzw. IIIa-IIIa in Fig. 1,
- Fig. 4:: eine Aufsicht auf das Flügelrad des ersten Ausführungsbeispiels aus der axialer Richtung (Deckplatte nicht dargestellt),
- Fig. 5:: einen Längsschnitt durch ein zweites Ausführungsbeispiel der erfindungsgemässen Zentrifugalpumpe,
- Fig. 6:: eine Detaildarstellung zur Verdeutlichung einer Variante für den Rotor der erfindungsgemässen Zentrifugalpumpe,
- Fig. 7:: eine Detaildarstellung zur Verdeutlichung einer weiteren Variante für den Rotor der erfindungsgemässen Zentrifugalpumpe,
- Fig. 8:: eine Seitenansicht eines Flügels des Flügelrads
- Fig. 9:: einen Längsschnitt durch ein erstes Ausführungsbeispiel der erfindungsgemässen Zentrifugalpumpenanordnung, und
- Fig. 10:: einen Längsschnitt durch ein zweites Ausführungsbeispiel der erfindungsgemässen Zentrifugalpumpenanordnung.

In der folgenden Beschreibung wird mit beispielhaftem Charakter auf den Anwendungsfall Bezug genommen, bei dem die erfindungsgemässe Zentrifugalpumpe bzw. Zentrifugalpumpenanordnung als Blutpumpe zur Übernahme der Herzfunktion eingesetzt wird. Es versteht sich jedoch, dass die erfindungsgemässe Zentrifugalpumpe bzw. Anordnung nicht auf solche Anwendungen beschränkt ist. Sie ist insbesondere auch zum Fördern anderer biologischer Flüssigkeiten, hochreiner Flüssigkeiten wie Reinstwasser, oder aggressiver Flüssigkeiten geeignet.

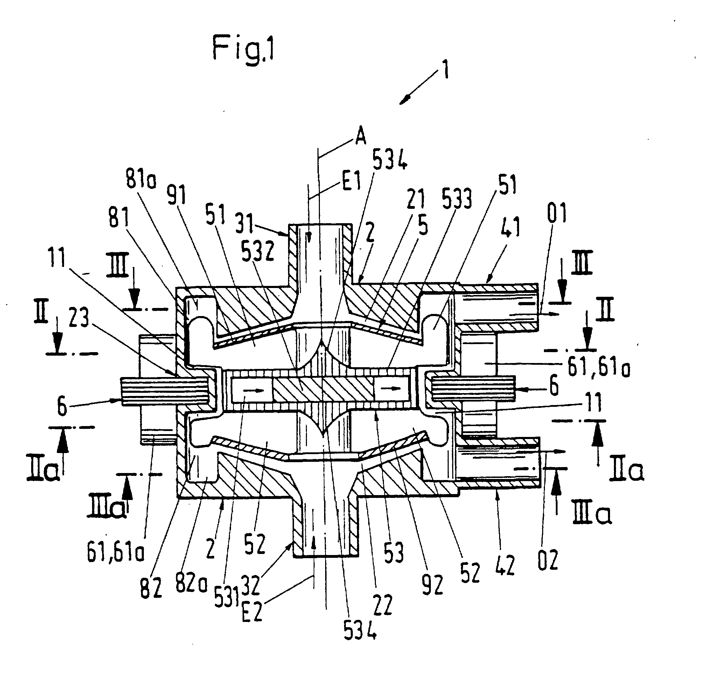

Die erfindungsgemässe Zentrifugalpumpe zum Fördern eines Fluids, insbesondere zum Fördern von Blut, hat ein Pumpengehäuse 2 (Fig. 1), das einen ersten und einen zweiten Einlass 31 bzw. 32 sowie einen ersten und einen zweiten Auslass 41 bzw. 42 für das Fluid aufweist. Im Innern des Pumpengehäuses 2 ist ein magnetisch lagerbares Flügelrad 5 angeordnet, das um eine Rotationsachse A rotierbar ist, und das einen magnetisch wirksamen, scheiben- oder ringförmigen Rotor 53 umfasst, sowie zwei Sätze von Flügeln 51 bzw. 52, wobei auf jeder der beiden axialen Begrenzungsflächen des Rotors 53 jeweils ein Satz von Flügeln 51 bzw. 52 vorgesehen ist. Die beiden Auslässe 41,42 sind in axialer Richtung zueinander versetzt angeordnet.

Mit Zentrifugalpumpen sind solche Rotations- oder Kreiselpumpen gemeint, bei denen das zu fördernde Fluid in Richtung der Rotationsachse A zu dem Flügelrad 5 strömt und das Pumpengehäuse 2 in radialer oder tangentialer Richtung verlässt.

Bei einer besonders bevorzugten Ausgestaltung der erfindungsgemässen Zentrifugalpumpe wird das Flügelrad 5 magnetisch und berührungslos im Pumpengehäuse 2 gelagert und durch ein elektomagnetisches Drehfeld angetrieben. Dazu ist der passive magnetisch wirksamen Rotor 53, der Teil des Flügelrads 5 ist, sowie ein das Pumpengehäuse 2 umgebenden Stator 6, der elektrische Wicklungen 61 aufweist, vorgesehen. Der Stator 6 ist vorzugsweise als Lager- und Antriebsstator ausgestaltet, sodass das Flügelrad 5 im Betriebszustand durch die magnetische Wechselwirkung zwischen dem Stator 6 und dem Rotor 53 sowohl antreibbar als auch berührungslos in dem Pumpengehäuse 2 lagerbar ist. Vorzugsweise bilden der Stator 6 und der Rotor 53 einen sogenannten lagerlosen Motor, der in analoger Weise ausgestaltet sein kann wie dies in der WO-A-96/31934 offenbart ist. Die Funktionsweise und die Ausgestaltung bezüglich Antrieb und magnetischer Lagerung des Rotors 53 sind in dieser Druckschrift, deren Inhalt hiermit in diese Beschreibung inkorporiert wird, detailiert beschrieben und werden daher hier nicht näher erläutert.

Im folgenden werden nur kurz die wesentlichsten Merkmale des Prinzips des lagerlosen Motors zusammengefasst. Unter einem lagerlosen Motor wird eine elektrisch ansteuerbare Lager- und Antriebsvorrichtung verstanden, welche den Stator 6 und den Rotor 53 umfasst. Der Begriff "lagerloser Motor" bringt zum Ausdruck, dass der Stator 6 gleichzeitig Antriebs- und Lagerstator ist, also kein separater Stator (und auch kein separater Rotor) für die magnetische Lagerung vorgesehen ist. Der Stator 6 ist so ausgestaltet bzw. mit elektrischen Wicklungen 61 versehen, dass er ein elektromagnetisches Drehfeld erzeugt, welches zum einen ein Drehmoment auf den Rotor 53 ausübt, dass dessen Rotation um die Rotationsachse A antreibt, und welches zum anderen eine beliebig einstellbare Querkraft auf den Rotor 53 ausübt, sodass dessen Position bezüglich einer zur Rotationsachse A senkrechten Ebene vorgebbar bzw. aktiv steuerbar ist. Somit ist der Rotor 53 im Betriebszustand mittels der elektrischen Wicklungen 61 des Stators 6 bezüglich dreier Freiheitsgrade, nämlich der Rotation um die Rotationsachse A sowie der Position bezüglich der zur Rotationsachse A senkrechten Ebene (zwei Freiheitsgrade) aktiv ansteuerbar bzw. antreibbar. Dazu umfassen die elektrischen Wicklungen 61 des Stators 6 beispielsweise eine Antriebswicklung mit der Polpaarzahl p und eine Steuerwicklung mit der Polpaarzahl p+1 oder p-1.

Bezüglich dreier weiterer Freiheitsgrade, nämlich Verkippungen bezüglich der zur Rotationsachse A senkrechten Ebene (zwei Freiheitsgrade) und axiale Position, ist der Rotor 53 vorzugsweise durch Reluktanzkräfte magnetisch passiv, das heisst nicht ansteuerbar, im Stator 6 gelagert. Dazu weist der Stator 6 z. B. mehrere radial nach innen weisende Zähne auf (nicht dargestellt). Für weitere Details der Ausgestaltung und der Ansteuerung des Stators 6 bzw. der Ausgestaltung des Rotors 53 sei hier auf die WO-A-96/31934 verwiesen. Die dort zu findenden Erläuterungen bezüglich Antrieb und Lagerung lassen sich in analoger Weise auch für die erfindungsgemässe Zentrifugalpumpe verwenden.

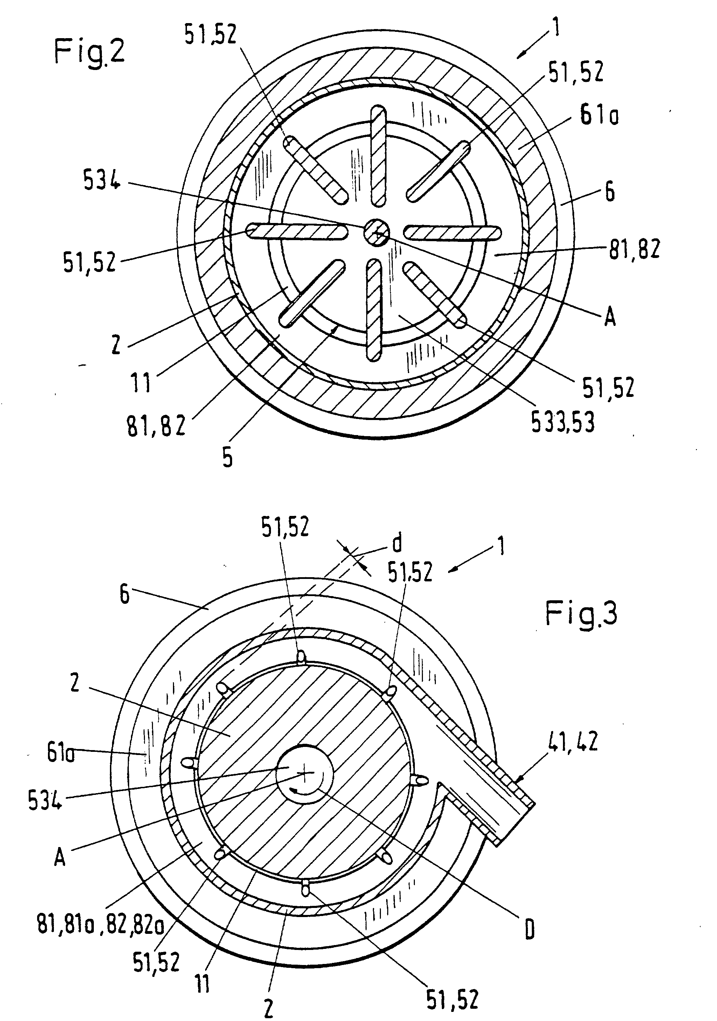

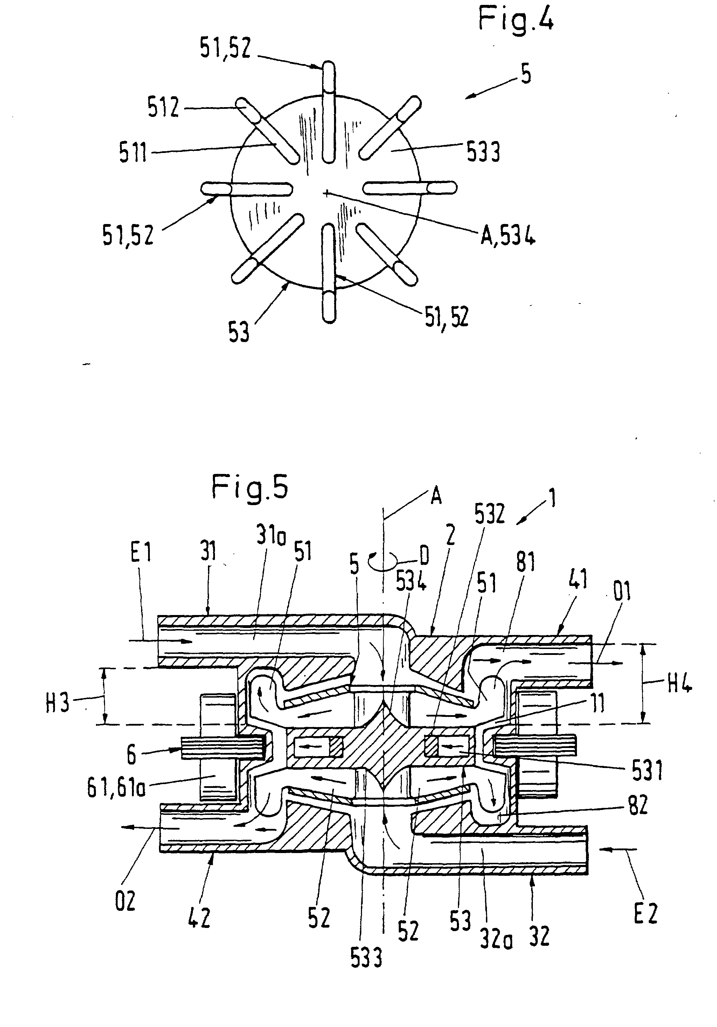

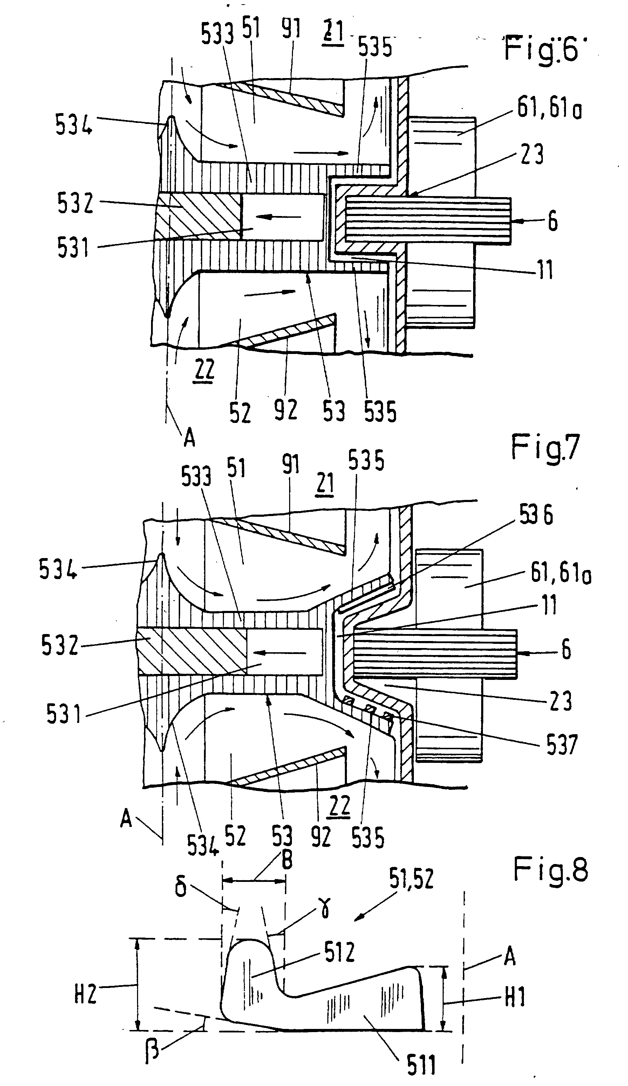

Im folgenden wird ein erstes Ausführungsbeispiel der erfindungsgemässen Zentrifugalpumpe, die gesamthaft mit dem Bezugszeichen 1 versehen ist, anhand der Fig. 1-4 sowie Fig. 8 näher erläutert. Fig. 1 zeigt das erste Ausführungsbeispiel in einer Längsschnittdarstellung. Zum besseren Verständnis zeigen die Figuren 2 und 3 dieses Ausführungsbeispiel jeweils noch in einer Querschnittsdarstellung. In Fig. 2 ist ein Querschnitt dargestellt, der sich sowohl für die Schnittlinie II-II als auch für die Schnittlinie IIa-IIa in Fig. 1 ergibt. Der in Fig. 3 dargestellte Querschnitt ergibt sich sowohl für die Schnittlinie III-III als auch für die Schnittlinie IIIa-IIIa in Fig. 1. Fig. 4 zeigt eine Aufsicht auf das Flügelrad 5 des ersten Ausführungsbeispiels aus der axialer Richtung. Eine weiter hinten erläuterte Deckplatte 91 bzw 92 (siehe Fig. 1, 6, 7) ist in Fig. 4 nicht dargestellt. Fig. 8 zeigt eine Seitenansicht eines Flügels 51 oder 52.

Das Pumpengehäuse 2 hat die beiden Einlässe 31, 32, die als axiale Einlässe angeordnet sind, und durch die das zu fördernde Fluid in das Pumpengehäuse gelangt wie dies die Pfeile E1 und E2 andeuten. Jeder der Einlässe 31,32 ist mit einem nicht dargestellten Fluidsysteme verbunden. Der erste Einlass 31 ist beispielsweise mit der Lungenvene verbunden, während der zweite Einlass 32 mit den Hohlvenen vom Körperkreislauf verbunden ist. Die beiden Auslässe 41,42 sind als tangentiale Auslässe ausgestaltet, durch die das Fluid das Pumpengehäuse 2 verlässt wie dies die Pfeile O1 und 02 andeuten. Auch die Auslässe 41,42 sind mit den Fluidsystemen verbunden. Der erste Auslass 41 ist beispielsweise mit der Aorta verbunden, während der zweite Auslass 42 mit der Lungenarterie verbunden ist.

Im Innern des Pumpengehäuses 2 ist das Flügelrad 5 angeordnet, das den hier scheibenförmigen Rotor 53 umfasst, sowie zwei Sätze von auf dem Rotor angeordneten Flügeln 51,52, die vorzugsweise aus Kunststoff hergestellt sind. Die zu dem ersten Satz gehörenden Flügel sind mit dem Bezugszeichen 51 versehen, die zu dem zweiten Satz gehörenden Flügel mit dem Bezugszeichen 52. Aus Gründen der besseren Übersicht sind in Fig. 1 für jeden Satz nur zwei der Flügel 51 bzw. 52 dargestellt.

Der scheibenförmige Rotor 53 umfasst einen ringförmigen, die Rotationsachse A umschliessenden Permanentmagnet 531, einen scheibenförmigen Eisenrückschluss 532, der das Loch des ringförmigen Permanentmagneten 531 ausfüllt, sowie eine Ummantelung 533, die vorzugsweise aus Titan oder Kunststoff hergestellt ist. Die Flügel 51,52 sind drehfest mit der Ummantelung 533 verbunden. Auf jeder der axialen Begrenzungsflächen des Rotors 53, also darstellungsgemäss auf seiner Ober- und auf seiner Unterseite ist jeweils ein Satz von Flügeln 51 bzw. 52 angeordnet. Gemäss der Darstellung in Fig. 1 ist der erste Satz Flügel 51 auf der Oberseite des Rotors 53 angeordnet und der zweite Satz Flügel 52 auf der Unterseite des Rotors 53. Der Permanentmagnet 531 ist beispielsweise diametral magnetisiert wie dies die beiden Pfeile ohne Bezugszeichen in seinem Innern andeuten.

Das Pumpengehäuse 2 ist etwa in seiner Mitte von dem Lager- und Antriebsstator 6 mit den elektrischen Wicklungen 61 umgeben. Die elektrischen Wicklungen 61 sind als Wickelköpfe 61a dargestellt. Der passive magnetisch wirksame Rotor 53 bildet zusammen mit dem Stator 6 und nicht dargestellten Versorgungs- und Steuereinrichtungen einen lagerlosen Motor, wobei der Rotor 53 bezüglich der Rotation um die Achse A sowie seiner Position in der zur Rotationsachse A senkrechten Ebene aktiv ansteuerbar bzw. antreibbar ist und hinsichtlich Verkippungen relativ zu genannter Ebene sowie seiner axialen Position magnetisch passiv durch Reluktanzkräfte im Stator 6 gelagert ist.

Wie insbesondere aus Fig. 1 ersichtlich, sind die beiden Auslässe erfindungsgemäss bezüglich der axialen Richtung versetzt zueinander angeordnet. Vorzugsweise ist der erste Auslass 41 darstellungsgemäss unmittelbar oberhalb und der zweite Auslass 42 darstellungsgemäss unmittelbar unterhalb der Wickelköpfe 61a angeordnet. Insbesondere liegen die Auslässe 41 bzw. 42 auf den Wickelköpfen 61a auf. Dies ermöglicht eine besonders kompakte und platzsparende Ausgestaltung der Zentrifugalpumpe 1. Durch den gegenseitigen Versatz der Auslässe 41, 42 kann die erfindungsgemässe Zentrifugalpumpe 1 die Funktion von zwei Pumpensystemen übernehmen. Im Betriebszustand rotiert das Flügelrad 5 um die Rotationsachse A. Der erste Satz Flügel 51 fördert somit das Fluid vom ersten Einlass 31 zum ersten Auslass 41 (erstes Pumpensystem), während der zweite Satz Flügel 52 das Fluid vom zweiten Einlass 32 zum zweiten Auslass 42 fördert (zweites Pumpensystem).

Bei dem hier beschriebenen Ausführungsbeispiel begrenzt das Flügelrad 5 im Innern des Pumpengehäuses 2 zwei im wesentlichen getrennte Pumpenkammern 21,22, nämlich die darstellungsgemäss obere erste Pumpenkammer 21 sowie die darstellungsgemäss untere zweite Pumpenkammer 22, wobei jeder Pumpenkammer 21, 22 jeweils einer der Einlässe 31,32 und einer der Auslässe 41,42 zugeordnet ist. Ein wesentlicher Aspekt ist hierbei, dass die Zentrifugalpumpe 1 zwei im wesentlichen getrennte Pumpensysteme realisiert, zwischen denen nur ein sehr geringer Fluidaustausch stattfindet. Das heisst, das vom ersten bzw. vom zweiten Einlass 31 bzw. 32 kommende Fluid wird hauptsächlich, beispielsweise zu mehr als 90%, zu dem zugeordneten ersten bzw. zweiten Auslass 41 bzw. 42 gefördert. Somit kann die erfindungsgemässe Zentrifugalpumpe die Funktion des Herzens übernehmen, indem z. B. das erste Pumpensystem die Funktion des linken Ventrikels (Körperkreislauf) und das zweite Pumpensystem die des rechten Ventrikels (Lungenkreislauf) übernimmt. Von besonderem Vorteil ist dabei die extrem kompakte und platzsparende Ausgestaltung der erfindungsgemässen Zentrifugalpumpe.

Jede der Pumpenkammern 21, 22 umfasst einen im wesentlichen ringförmigen, in Umfangsrichtung des Pumpengehäuses verlaufenden Leitkanal 81 bzw. 82, der den der Pumpenkammer 21 bzw. 22 zugeordneten Einlass 31 bzw. 32 mit dem der gleichen Pumpenkammer 21 bzw.22 zugeordneten Auslass 41 bzw. 42 verbindet. Dabei umfasst jeder Leitkanal 81, 82 einen Teilraum 81a, 82a der in axialer Richtung gesehen bezüglich des Flügelrads 5 versetzt angeordnet ist. Das heisst, die Leitkanäle 81 und 82 sind in die axiale Richtung gefaltet. Darstellungsgemäss (siehe Fig. 1) ist der Leitkanal 81 der ersten Pumpenkammer 21 nach oben gefaltet und der Leitkanal 82 der zweiten Pumpenkammer 22 ist nach unten gefaltet.

Da somit die Leitkanäle 81,82 durch die axial versetzten Teilräume 81a,82a jewils ausreichend Volumen für das zu fördernde Fluid aufweisen können als weitere vorteilhafte Massnahme die Flügel 51,52 des Flügelrads 5 so ausgestaltet werden, dass sie sich in radialer Richtung gesehen jeweils bis in den ihnen zugeordneten Leitkanal 81 bzw. 82 hinein erstrecken. Durch diese Massnahme vergrössert sich nämlich die effektiv wirksame Flügellänge, wodurch sich die Leistungsfähigkeit, insbesondere die Förderleistung, der Zentrifugalpumpe 1 noch steigern lässt, ohne dass sich die äusseren Abmessungen der Zentrifugalpumpe 1 vergrössern.

Besonders bevorzugt sind die Flügel 51, 52 jeweils gewinkelt ausgestaltet (siehe Fig. 1 und Fig. 8), sodass sie jeweils einen Radialteil 511 aufweisen, der sich geradlinig oder gekrümmt in radialer Richtung nach aussen erstreckt, sowie einen daran anschliessenden Axialteil 512, der sich im wesentlichen parallel zur Rotationsachse A des Flügelrads 5 bzw. parallel zur Seitenwand des Pumpengehäuses 2 ersreckt. Der Axialteil 512 reicht bis in den Teilraum 81a oder 82a des Leitkanals 81 oder 82 hinein. Die Flügel 51,52 sind also jeweils in die axiale Richtung gefaltet. Gemäss der Darstellung in Fig. 1 sind die Flügel 51 des ersten Satzes nach oben gefaltet und die Flügel 52 des zweiten Satzes nach unten. Durch diese vorteilhafte Massnahme wird die effektiv wirksame Flügellänge noch weiter vergrössert, wodurch sich die Leistungsfähigkeit, insbesondere die Förderleistung, der Zentrifugalpumpe nochmals steigern lässt, ohne dass sich die äusseren Abmessungen der Zentrifugalpumpe vergrössern. Insbesondere wird durch die gefaltete Ausgestaltung der Flügel 51,52 bewirkt, dass das Fluid länger durch die Flügel 51,52 geführt wird, wodurch sich der Druck des Fluids erhöhen lässt.

Besonders vorteilhaft ist, dass mehrere Möglichkeiten bestehen, sowohl das erste Pumpensystem (erster Einlass 31, erster Satz Flügel 51 erster Leitkanal 81, erster Auslass 41) als auch das zweite Pumpensystem (zweiter Einlass 32, zweiter Satz Flügel 52, zweiter Leitkanal 82, zweiter Auslass 42) jeweils für sich hydraulisch an die zu übernehmende Funktion anzupassen. Bei der Ausgestaltung als Blutpumpe, insbesondere als künstliches Herz, ist es beispielsweise aus physiologischen Gründen notwendig, dass das Pumpensystem, welches den linken Ventrikel (Körperkreislauf) ersetzt, einen höheren Druck erzeugt als das den rechten Ventrikel (Lungenkreislauf) ersetzende Pumpensystem. Eine solche hydraulische Anpassung ist bei der erfindungsgemässen Zentrifugalpumpe 1 beispielsweise über eine unterschiedliche Ausgestaltung der Flügel 51 des ersten Satzes einerseits und der Flügel 52 des zweiten Satzes möglich. Dazu können die Flügel 51 des einen Satzes eine andere effektive Flügellänge aufweisen als die Flügel 52 des anderen Satzes. Beispielsweise können die Flügel 51 des ersten Satzes in radialer Richtung gesehen weiter nach aussen reichen als die Flügel 52 des zweiten Satzes oder umgekehrt. Auch ist es möglich, wie aus Fig. 1 ersichtlich, dass der Axialteil 512 der Flügel 51 im Vergleich zu den Flügeln 52 länger ausgestaltet ist, also eine grössere axiale Höhe H2 (siehe Fig. 8) aufweist und somit weiter in den Teilraum 81a hineinreicht oder umgekehrt. Auch hierdurch lassen sich unterschiedliche effektive Flügellängen für die beiden Sätze von Flügel 51, 52 realisieren. Gemäss der Darstellung in Fig. 1 wird das erste (obere) Pumpensystem, das die Flügel 51 mit den längeren Axialteilen 512 (grössere Höhe H2) aufweist, bei gleichem Durchfluss und vorgegebener Drehzahl des Flügelrads 5 einen höheren Druck am ersten Auslass 41 erzeugen als das zweite (untere) Pumpensystem am zweiten Auslass 42. Bei der Anwendung als künstliches Herz wird somit das erste (obere) Pumpensystem vorzugsweise den linken Ventrikel ersetzen und das zweite (untere) Pumpensystem den rechten Ventrikel.

Ferner ist die individuelle hydraulische Anpassung der beiden Pumpensysteme auch über die Form, z. B. die axiale Höhe H1 (Fig. 8), der Radialteile 511 der Flügel 51, 52 möglich, sowie über die Breite B der Axialteile 512 in radialer Richtung, und über die geometrische Ausgestaltung der Pumpenkammern 21,22, insbesondere der Leitkanäle 81, 82.

Weiterhin sind Ausgestaltungen des Flügelrads 5 möglich, bei denen die Anzahl der Flügel 51 des ersten Satzes verschieden ist von der Anzahl der Flügel 52 des zweiten Satzes. Auch ist es möglich die Flügel 51 des einen Satzes gewinkelt auszugestalten und die Flügel 52 des anderen Satzes nicht-gewinkelt, also ohne Axialteil 512, oder umgekehrt.

Wie dies am besten aus Fig. 2 und Fig. 3 ersichtlich ist, können die Leitkanäle 81,82 jeweils als Ringraum ausgestaltet sein, das heisst mit einem Querschnitt, der über ihren Umfang im wesentlichen gleichbleibend ist, sodass alle radial äussern Enden der Flügel 51 oder 52 im normalen Betriebszustand im wesentlichen den gleichen Abstand d (Fig. 3) zur Seitenwand des Pumpengehäuses 2 haben.

Alternativ können die Leitkanäle 81, 82 in radialer Richtung jeweils als Spiralraum ausgestaltet sein, sodass der Abstand d zwischen den radial äusseren Enden der Flügel 51,52 und der die Leitkanäle 81, 82 begrenzenden Seitenwand des Pumpengehäuses 2 in Rotationsrichtung gesehen zunimmt. Die Rotationrichtung ist in Fig. 3 durch den Pfeil D angedeutet. Der Abstand d ist dann jeweils im Bereich des kürzeren Schenkels des tangentialen Auslasses 41 bzw. 42 am geringsten und nimmt in Rotationsrichtung D gesehen zu. Diese Ausgestaltung der Leitkanäle 81,82 als radiale Spiralräume trägt der Tatsache Rechnung, dass das Volumen des im jeweiligen Leitkanal 81,82 befindlichen Fluids in Rotationsrichtung D gesehen zum Auslass 41, 42 hin zunimmt.

In axialer Richtung kann zwischen den Flügeln 51 bzw. 52 des Flügelrads 5 und der ihnen zugewandten inneren Wand des Pumpengehäuses 2 noch jeweils eine konische Deckplatte 91 bzw. 92 (siehe Fig. 1, 6, 7) angeordnet sein, z. B. jeweils auf die Flügel 51 bzw. 52 aufgesetzt werden, um die Rückstömung des Fluids aus dem jeweiligen Leitkanal 81 bzw. 82 in Richtung des zugehörigen Einlasses 31 bzw. 32 zu reduzieren. Die Deckplatten 91,92 erstrecken sich in radialer Richtung ungefähr bis an die radial innere Begrenzung des jeweiligen Leitkanals 81 bzw. 82.

Um eine gute Flussführung in den beiden Pumpensystemen zu gewährleisten, weist der Rotor 53 vorzugsweise Mittel zur Flussführung des Fluids auf. Diese Mittel umfassen beispielsweise (siehe Fig. 1) zwei kegelförmige Erhebungen 534 im zentralen Bereich der Ummantelung 533 des Rotors 53, wobei die Spitzen der kegelförmigen Erhebungen 534 im Betriebszustand jeweils ungefähr auf der Rotationsachse A liegen. Die eine kegelförmige Erhebung 534 erstreckt sich darstellungsgemäss nach oben, also in Richtung des ersten Einlasses 31, während sich die zweite kegelförmige Erhebung 534 darstellungsgemäss nach unten, also in Richtung des zweiten Einlasses 32, erstreckt.

Das Pumpengehäuse 2 ist auf seiner Aussenseite in seinem bezüglich der Achse A mittleren Bereich mit einer Umfangsnut 23 versehen, die den Innenraum des Pumpengehäuses 2 in demjenigen axialen Bereich verengt, in welchem im Betriebszustand der Rotor 53 rotiert. In diese Umfangsnut 23 hinein erstreckt sich der Stator 6. Durch diese Massnahme ist zum einen gewährleistet, dass der Stator 6 möglichst nahe an dem Rotor 53 angeordnet ist, was im Hinblick auf den Antrieb und die magnetische Lagerung des Rotors 53 vorteilhaft ist, zum anderen dient die Verengung des Inneren des Pumpengehäuses 2 als Trennung zwischen den beiden Pumpenkammern 21 und 22 bzw. zwischen den beiden Leitkanälen 81 und 82. Die beiden Leitkanäle 81 und 82 stehen somit nur noch über einen sehr engen Spalt 11 in Verbindung, durch den im Betriebszustand nur ein sehr geringfügiger, für viele Anwendungen nicht störender, Fluidaustausch zwischen den beiden Pumpensystemen der Zentrifugalpumpe 1 möglich ist.

Im Betriebszustand rotiert das Flügelrad 5, angetrieben und in der Schwebe gehalten durch die magnetische Wechselwirkung zwischen dem Rotor 53 und dem Lager- und Antriebsstator 6, um die Rotationsachse A. Dadurch fördern sowohl das erste als auch das zweite Pumpensystem Fluid und zwar jeweils im wesentlichen nur von dem jeweiligen Einlass 31 bzw. 32 zu dem ihm zugeordneten Auslass 41 bzw. 42. Vorteilhaft ist dabei, dass das Flügelrad beidseitig (bezüglich der axialen Richtung) von dem Fluid beaufschlagt wird bzw. dieses fördert. Hieraus resultiert nämlich eine zumindest teilweise Kompensation der Axialschübe auf das Flügelrad 5, wodurch sich die Axialkräfte auf das Flügelrad 5 gegenseitig zumindest teilweise aufheben. Somit reicht die passive magnetische Axiallagerung des Flügelrads 5 insbesondere auch bei hohen Förderleistungen aus.

Fig. 5 zeigt in einer Längsschnittdarstellung ein zweites Ausführungsbeispiel der erfindungsgemässen Zentrifugalpumpe 1. Auch in Fig. 5 sind aus Gründen der besseren Übersicht nur jeweils zwei der Flügel 51 bzw. 52 dargestellt. Im folgenden werden nur die Unterschiede zum ersten Ausführungsbeispiel beschrieben. Ansonsten gelten die Erläuterungen des ersten Ausführungsbeispiels in sinngemäss gleicher Weise auch für das zweite Ausführungsbeispiel.

Bei dem zweiten Ausführungsbeispiel nimmt die axiale Höhe H3, H4 jedes Leitkanals 81,82 in Umfangsrichtung zu, sodass jeder Leitkanal 81,82 einen axialen Spiralraum bildet. Die axiale Höhe H3,H4 des Leitkanals 81 bzw. 82 hat in Rotationsrichtung - angedeutet durch den Pfeil D-gesehen unmittelbar hinter dem Auslass 41 bzw. 42 ihren minimalen Wert und nimmt dann entlang der Umfangsrichtung kontinuierlich zu. An der dem Auslass 41 bzw. 42 gegenüberliegenden Seite hat der Leitkanal 81 bzw. 82 z. B. die axiale Höhe H3. Die axiale Höhe nimmt dann in Rotationsrichtung D gesehen weiter zu und erreicht am Auslass 41 bzw. 42 ihren maximalen Wert H4 (H4 > H3).

Die Ausgestaltung der Leitkanäle 81, 82 als axiale Spiralräume ist unter stömungstechnischen Aspekten besonders vorteilhaft, insbesondere in Kombination mit den gewinkelten Flügeln 51, 52.

Es versteht sich, dass auch solche Ausgestaltungen möglich sind, bei denen die Leitkanäle 81, 82 sowohl als radiale als auch als axiale Spiralräume ausgestaltet sind, sich also in Umfangsrichtung betrachtet radial erweitern und gleichzeitig eine zunehmende axiale Höhe aufweisen.

Auch ist es möglich, die beiden Leitkanäle 81, 82 unterschiedlich auszugestalten.

Ein weiterer Unterschied bei dem zweiten Ausführungsbeispiel besteht in der Ausgestaltung der Einlässe 31, 32. Jeder Einlass 31,32 umfasst einen gewinkelten Einlasskanal 31a bzw. 32a, der sich zunächst im wesentlichen radial zur Rotationsachse A hin erstreckt, und sich dann in die Richtung der Rotationsachse A krümmt, sodass das Fluid jeweils aus der axialen Richtung zu dem Flügelrad 5 gelangen kann, wie dies die Pfeile E1 bzw. E2 in Fig. 5 andeuten. Wie dies Fig. 5 zeigt, ist jeweils der radial verlaufende Teil des Einlasskanals 31a bzw. 32a von einer anderen Seite zur Rotationsachse A geführt als die Seite, zu der sich der zugeordnete Auslass 41 bzw. 42 erstreckt. Diese Massnahme erlaubt insbesondere in Kombination mit der Ausgestaltung des Leitkanals 81 bzw. 82 als axialer Spiralraum eine besonders kompakte Ausgestaltung der Zentrifugalpumpe 1. Da nämlich der Leitkanal 81 bzw. 82 auf der dem zugehörigen Auslass 41 bzw. 42 gegenüberliegenden Seite eine geringere axiale Höhe H3 aufweist als auf der Seite, wo der zugehörige Auslass 41 bzw. 42 angeordnet ist (axiale Höhe H4), kann der radial verlaufende Teil des zugehörigen Einlasskanals 31a bzw. 32a bezüglich der axialen Richtung näher am Flügelrad 5 angeordnet werden, insbesondere derart, dass sich der radial verlaufende Teil des Einlasskanals 31a bzw. 32a und der zugeordnete Auslass 41 bzw. 42 bezüglich ihrere axialen Positionen zumindest teilweise überlappen. Vorzugsweise sind die Einlasskanäle 31a, 32a in das Pumpengehäuse 2 integriert. Durch diese Massnahmen erhält die Zentrifugalpumpe 1 aufgrund der optimierten Ausnutzung des zur Verfügung stehenden Platzes eine möglichst geringe Ausdehnung in axialer Richtung., wodurch sie sehr kompakt ist und zudem eine hohe Förderleistung ermöglicht.

Ferner sind bei dem zweiten Ausführungsbeispiel die beiden Auslässe 41, 42 bezüglich der Umfangsrichtung des Pumpengehäuses 2 versetzt, darstellungsgemäss um etwa 180°, angeordnet. Dies hat insbesondere für Blutpumpen den Vorteil, dass die beiden Pumpensysteme der Zentrifugalpumpe 1 beim Einbau in den Körper besser mit den jeweiligen Blutgefässen, nämlich den Hohlvenen und der Lungenarterie bzw. der Lungenvene und der Aorta, verbunden werden können. Die gegenseitige Versetzung der Auslässe 41,42 bzw. der Einlässe 31,32 um 180° ist natürlich nur beispielhaft zu verstehen. Es sind durchaus auch andere Winkel möglich. Insbesondere kann die Lage der Ein- und Auslässe 31,32,41,42 an die physiologischen Gegebenheiten oder individuell an den Patienten angepasst werden.

Bei dem zweiten Ausführungsbeispiel ist ferner der Eisenrückschluss 532 im Rotor nicht als Scheibe sondern als Ring ausgestaltet, der konzentrisch und radial innenliegend zum Permanentmagnet 531 angeordnet ist.

Im folgenden werden noch einige vorteilhafte konstruktive Varianten beschrieben, die für beide Ausführungsbeispiele Verwendung finden können.

Die Detaildarstellung in Fig. 6 verdeutlicht eine Variante der Ausgestaltung des Rotors 53. Hier weist die Ummantelung 533 des Rotors 53 an ihrem radial äusseren Ende zwei Fortsätze 535 auf, die sich darstellungsgemäss ober- und unterhalb der Umfangsnut 23 erstrecken. Durch diese Massnahme wird der die beiden Pumpenkammern 21 und 22 verbindende Spalt 11 verlängert und es lässt sich ein Labyrinthdichtungseffekt erzielen. Hieraus resultiert eine noch bessere Abdichtung der beiden Pumpenkammern 21 und 22 gegeneinander, wodurch sich der Fluidaustausch zwischen den beiden Pumpensystemen weiter reduzieren lässt.

Bei der durch Fig. 7 veranschaulichten Variante verlaufen die Fortsätze 535 der Ummantelung 533 bezüglich der radialen Richtung schräg, also schiefwinklig bezüglich der Rotationsachse A. Der darstellungsgemäss obere Fortsatz 535 verläuft schräg nach oben, der darstellungsgemäss untere Fortsatz 535 verläuft schräg nach unten. Vorzugsweise ist die Umfangsnut 23 so ausgestaltet, dass sie der Abschrägung der Fortsätze 535 folgt. Die Abschrägung der Fortsätze 535 sind ein weiteres Mittel zur Verbesserung der Flussführung.

Ferner kann es vorteilhaft sein, an der dem Spalt 11 zugewandten Seite der Fortsätze 535 Zusatzflügel 536 und/oder spiralförmige Erhebungen 537 oder spiralförmige Vertiefungen vorzusehen. Diese dienen als Mittel zur Vermeidung von Fluidstagnationen. Durch die Zusatzflügel 536 bzw. Die spiralförmige Erhebung 537 oder Vertiefung kann erreicht werden, dass auch das im Spalt 11 befindliche Fluid gefördert wird. Insbesondere bei Blutpumpen lässt sich hierdurch die Gefahr der Trombenbildung vermeiden Die Mittel zur Vermeidung von Fluidstagnationen 536, 537 können entweder an beiden Fortsätzen 535 oder nur an einem der Fortsätze 535 vorgesehen sein.

Fig. 8 zeigt eine Variante für die Flügel 51, 52 in einer Detaildarstellung. Bei dieser Ausgestaltung ist das radial äussere Ende des Flügels 51, 52 auf seiner dem zugehörigen Einlass 31 bzw. 32 abgewandten Seite - gemäss der Darstellung in Fig. 8 ist dies die Unterseite des Flügels 51,52 - bezüglich der Rotationsebene, die senkrecht zur Rotationsachse A verläuft, unter einem Winkel β abgeschrägt ausgestaltet. Hierdurch wird vermieden, dass die Flügel 51,52 bei geringfügigen Verkippungen des Flügelrads 5 an das Pumpengehäuse 2 anschlagen. Vorzugsweise sind auch die Axialteile 512 der Flügel 51,52 an ihren radial inneren und/oder radial äusseren Begrenzungen unter einem Winkel γ bzw. δ abgeschrägt bezüglich der Richtung der Rotationsachse A ausgestaltet, um ein Anschlagen der Flügel 51, 52 bei geringfügigen Verkippungen bezüglich der Rotationsebene zu vermeiden. Aus praktischen Gründen sind die Winkel β, γ, und δ der Abschrägungen vorzugsweise jeweils kleiner als 10° und speziell kleiner als 5°. Insbesondere sind solche Ausgestaltungen bevorzugt, bei denen alle drei Winkel β, γ und δ den gleichen Wert haben.

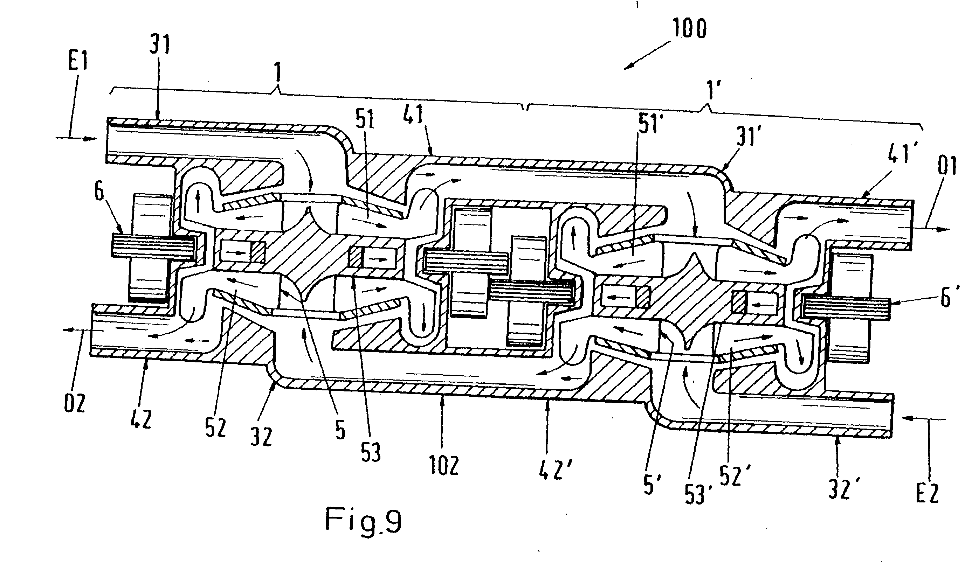

Fig. 9 zeigt einen Längsschnitt durch ein erstes Ausführungsbeispiel der erfindungsgemässen Zentrifugalpumpenanordnung, die gesamthaft mit dem Bezugszeichen 100 versehen ist. Die Zentrifugalpumpenanordnung 100 umfasst mindestens zwei Zentrifugalpumpen 1 und 1'. Die Bezugszeichen haben die gleiche Bedeutung wie sie bereits im Zusammenhang mit der Zentrifugalpumpe 1 erläutert wurden. Zur Unterscheidung der beiden Zentrifugalpumpen 1 und 1' sind die Bezugszeichen, die sich auf die Zentrifugalpumpe 1' beziehen, jeweils mit einem Strich versehen, bezeichnen aber jeweils das gleiche Element wie das entsprechende Bezugszeichen ohne Strich. Die Zentrifugalpumpenanordnung 100 (Fig. 9) umfasst die zwei Zentrifugalpumpen 1 und 1', die jeweils gemäss dem zweiten Ausführungsbeispiel (Fig. 5) ausgestaltet sind. Als Einlässe der Anordnung 100 dienen der erste Einlass 31 der ersten Zentrifugalpumpe 1 sowie der zweite Einlass 32' der zweiten Zentrifugalpumpe 1'. Als Auslässe der Anordnung 100 dienen der erste Auslass 41' der zweiten Zentrifugalpumpe 1' sowie der zweite Auslass 42 der ersten Zentrifugalpumpe 1. Der erste Auslass 41 der ersten Zentrifugalpumpe 1 ist mit dem ersten Einlass 31' der zweiten Zentrifugalpumpe 1' verbunden und der zweite Ausgang 42' der zweiten Zentrifugalpumpe 1' mit dem zweiten Eingang 32 der ersten Zentrifugalpumpe 1. Auf diese Weise sind die Zentrifugalpumpen 1,1' derart miteinander verbunden, dass sie eine hydraulische Serienschaltung bilden.

Es ist möglich, die beiden Zentrifugalpumpen 1,1' in einem gemeinsamen Pumpengehäuse 102 anzuordnen.

Eine solche Anordnung 100 ist insbesondere für solche Anwendungen vorteilhaft, bei denen es auf eine möglichst hohe Betriebssicherheit ankommt. Insbesondere ist eine solche Anordnung auch als Blutpumpe, speziell als künstliches Herz, geeignet. Falls eine der beiden Zentrifugalpumpen 1,1' ausfällt, gewährleistet die andere noch den ordnungsgemässen Betrieb der Anordnung, denn jede der Zentrifugalpumpen 1 und 1' alleine ist von ihrer Förderleistung und aufgrund des erfindungsgemässen Merkmals, zwei Pumpensysteme zu realisieren, in der Lage, die Funktion des Herzens zu übernehmen. Die durch die Hintereinanderschaltung realisierte Redundanz bedeutet somit eine ganz erhebliche Steigerung der Betriebssicherheit. Zudem ermöglicht die äusserst kompakte Ausgestaltung der erfindungsgemässen Zentrifugalpumpe 1,1' auch eine sehr platzsparende Ausführung der Anordnung 100. Die gesamte Anordnung 100 kann beispielsweise so ausgestaltet werden, dass ihre axiale Höhe etwa 45 mm beträgt und ihre Ausdehnung in den radialen Richtungen 120 mm auf 60 mm nicht überschreitet. Trotz dieser äusserst kompakten Ausgestaltung ermöglicht jede der Zentrifugalpumpen 1, 1' eine maximale Förderleistung, die über der des menschlichen Herzens liegt.

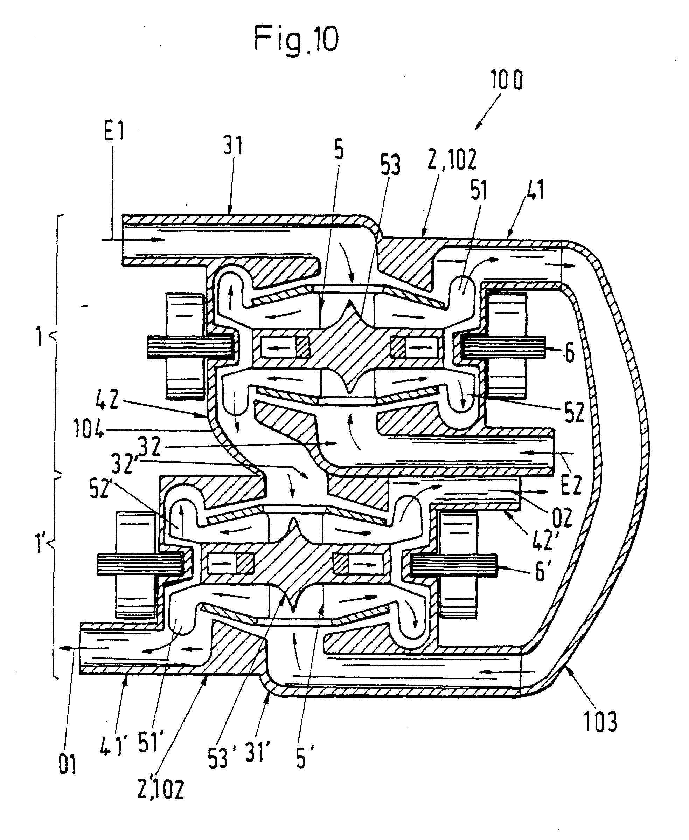

Fig. 10 zeigt einen Längsschnitt durch ein zweites Ausführungsbeispiel der erfindungsgemässen Zentrifugalpumpenanordnung 100. Auch hier sind zwei Zentrifugalpumpen 1 und 1' vorgesehen, die hydraulisch in Serie geschaltet sind. Bei der Darstellung in Fig. 10 steht die zweite (untere) Zentrifugalpumpe 1' auf dem Kopf (bezüglich der Darstellung in Fig. 5), d. h. bei ihr ist der erste Einlass 31' und der erste Auslass 41' darstellungsgemäss unten. Als Einlässe der Anordnung 100 dienen hier die beiden Einlässe 31 und 32 der ersten Zentrifugalpumpe 1. Als Auslässe der Anordnung 100 dienen die beiden Auslässe 41' und 42' der zweiten Zentrifugalpumpe 1'. Der erste Auslass 41 der ersten Zentrifugalpumpe 1 ist mit dem ersten Einlass 31' der zweiten Zentrifugalpumpe 1' verbunden, wie dies symbolisch durch die Schlauchleitung 103 angedeutet ist. Der zweite Auslass 42 der ersten Zentrifugalpumpe 1 ist mit dem zweiten Einlass 32' der zweiten Zentrifugalpumpe 1' verbunden, wie dies symbolisch durch die Verbindung 104 angedeutet ist.

Bei einer Ausgestaltung der Zentrifugalpumpenanordnung 100 (Fig. 9, Fig. 10) als Blutpumpe ist beispielsweise der erste Einlass der Anordnung 100 (erster Einlass 31 der ersten Zentrifugalpumpe 1) mit den Hohlvenen verbunden, der zweite Einlass der Anordnung 100 (zweiter Einlass 32' der zweiten Zentrifugalpumpe 1' bzw. zweiter Einlass 32 der ersten Zentrifugalpumpe 1) mit der Lungenvene, der erste Auslass der Anordnung 100 (erster Auslass 41' der zweiten Zentrifugalpumpe 1') mit der Aorta und der zweite Auslass der Anordnung 100 (zweiter Auslass 42 der ersten Zentrifugalpumpe 1 bzw. zweiter Auslass 42' der zweiten Zentrifugalpumpe 1') mit der Lungenarterie.

Natürlich sind auch solche Zentrifugalpumpenanordnungen möglich, bei denen die Zentrifugalpumpen hydraulisch parallel geschaltet sind. Prinzipiell ist die Erzielung der Redundanz durch das Zusammenschalten zweier Pumpen nicht auf die hier beschriebenen Pumpen beschränkt.

Die erfindungsgemässe Zentrifugalpumpe zeichnet sich insbesondere dadurch aus, dass sie zwei im wesentlichen getrennte Pumpensysteme in einer Pumpe realisiert, dabei extrem kompakt und platzsparend ausgestaltet ist und zudem eine hohe Förderleistung ermöglicht, deren Maximalwerte über der Förderleistung des menschlichen Herzens liegt. Die erfindungsgemässe Zentrifugalpumpe ist dadurch auch als Blutpumpe, insbesondere als künstliches Herz, für Anwendungen innerhalb und ausserhalb des Körpers geeignet. Durch die erfindungsgemässe Zentrifugalpumpenanordnung wird eine sehr sichere Anordnung bereitgestellt, die ebenfalls die Funktion des Herzens innerhalb oder ausserhalb des Körpers übernehmen kann.

## Patentansprüche

1. Zentrifugalpumpe zum Fördern eines Fluids, insbesondere zum Fördern von Blut, mit einem Pumpengehäuse (2), das einen ersten und einen zweiten Einlass (31,32) sowie einen ersten und einen zweiten Auslass (41,42) für das Fluid aufweist, mit einem im Innern des Pumpengehäuses (2) angeordneten, magnetisch gelagerten Flügelrad (5), das um eine Rotationsachse (A) rotierbar ist, und das einen magnetisch wirksamen, scheiben- oder ringförmigen Rotor (53) umfasst, sowie zwei Sätze von Flügeln (51,52), wobei auf jeder der beiden axialen Begrenzungsflächen des Rotors (53) jeweils ein Satz von Flügeln (51,52) vorgesehen ist, **dadurch gekennzeichnet, dass** die beiden Auslässe (41,42) in axialer Richtung zueinander versetzt angeordnet sind.

2. Zentrifugalpumpe nach Anspruch 1, bei der das Flügelrad (5) im Innern des Pumpengehäuses (2) zwei im wesentlichen getrennte Pumpenkammern (21,22) begrenzt, und jeder Pumpenkammer (21,22) jeweils einer der Einlässe (31,32) und einer der Auslässe (41,42) zugeordnet ist.

3. Zentrifugalpumpe nach Anspruch 2, wobei jede Pumpenkammer (21,22) einen im wesentlichen ringförmigen, in Umfangsrichtung des Pumpengehäuses verlaufenden Leitkanal (81,82) umfasst, der den der Pumpenkammer (21,22) zugeordneten Einlass (31,32) mit dem der gleichen Pumpenkammer (21,22) zugeordneten Auslass (41,42) verbindet, wobei jeder Leitkanal (81,82) einen Teilraum (81a,82a) umfasst, der in axialer Richtung gesehen bezüglich des Flügelrads (5) versetzt angeordnet ist.

4. Zentrifugalpumpe nach einem der vorangehenden Ansprüche mit einem das Pumpengehäuse (2) umgebenden Stator (6), der elektrische Wicklungen (61) aufweist, wobei der Stator (6) als Lager- und Antriebsstator ausgestaltet ist, sodass das Flügelrad (5) im Betriebszustand durch die magnetische Wechselwirkung zwischen dem Stator (6) und dem Rotor (53) sowohl antreibbar als auch berührungslos in dem Pumpengehäuse (2) lagerbar ist.

5. Zentrifugalpumpe nach Anspruch 4, wobei der Rotor (53) im Betriebszustand mittels der elektrischen Wicklungen (61) des Stators (6) bezüglich dreier Freiheitsgrade aktiv ansteuerbar bzw. antreibbar ist und bezüglich dreier weiterer Freiheitsgrade durch Reluktanzkräfte magnetisch passiv im Stator (6) gelagert ist.

6. Zentrifugalpumpe nach einem der vorangehenden Ansprüche, wobei die Flügel (51,52) jeweils gewinkelt ausgestaltet sind, sodass sie jeweils einen Radialteil (511) aufweisen, der sich geradlinig oder gekrümmt in radialer Richtung nach aussen erstreckt, sowie einen daran anschliessenden Axialteil (512), der sich im wesentlichen parallel zur Rotationsachse (A) des Flügelrads (5) ersreckt.

7. Zentrifugalpumpe nach einem der vorangehenden Ansprüche, wobei die Flügel (51) des einen Satzes eine andere effektive Flügellänge aufweisen als die Flügel (52) des anderen Satzes.

8. Zentrifugalpumpe nach einem der vorangehenden Ansprüche, wobei die beiden Auslässe (41,42) bezüglich der Umfangsrichtung des Pumpengehäuses (2) versetzt angeordnet sind.

9. Zentrifugalpumpe nach einem der vorangehenden Anspruche, wobei die axiale Höhe (H3,H4) jedes Leitkanals (81,82) in Umfangsrichtung zunimmt.

10. Zentrifugalpumpe nach einem der vorangehenden Ansprüche, wobei jeder Einlass (31,32) einen gewinkelten Einlasskanal (31a,32a) umfasst, der sich zunächst im wesentlichen radial zur Rotationsachse (A) hin erstreckt und sich dann in die Richtung der Rotationsachse (A) krümmt, sodass das Fluid aus der axialen Richtung zu dem Flügelrad (5) gelangen kann.

11. Zentrifugalpumpe nach einem der vorangehenden Ansprüche, wobei der Rotor (53) Mittel (534;535) zur Flussführung des Fluids aufweist.

12. Zentrifugalpumpe nach einem der vorangehenden Ansprüche, wobei Mittel (536;537) zur Vermeidung von Fluidstagnationen vorgesehen sind.

13. Zentrifugalpumpenanordnung mit mindestens zwei Zentrifugalpumpen (1,1'), von denen jede nach einem der vorangehenden Ansprüche ausgebildet ist.

14. Zentrifugalpumpenanordnung nach Anspruch 13, wobei die Zentrifugalpumpen (1,1') derart miteinander verbunden sind, dass sie eine hydraulische Serienschaltung bilden.

15. Blutpumpe, insbesondere künstliches Herz, mit einer Zentrifugalpumpe (1) nach einem der Ansprüche 1-12 oder einer Zentrifugalpumpenanordung (100) nach einem der Ansprüche 13-14.

## Claims

1. Centrifugal pump for delivering a fluid, especially for delivering blood, with a pump housing (2) that has a first and a second inlet (31, 32) as well as a first and a second outlet (41, 42) for the fluid, with a magnetically mounted impeller (5) located inside pump housing (2), said impeller being rotatable around a rotation axis (A) and comprising a magnetically effective rotor (53) in the shape of a disk or ring, as well as two sets of vanes (51, 52), with a set of vanes (51, 52) being provided on each of the two axial boundary surfaces of rotor (53), **characterized in that** the two outlets (41, 42) are offset with respect to one another in the axial direction.

2. Centrifugal pump according to Claim 1, in which impeller (5) delimits two essentially separate pump chambers (21, 22) in the interior of pump housing (2), and each pump chamber (21, 22) is associated with one of inlets (31, 32) and one of outlets (41, 42).

3. Centrifugal pump according to Claim 2, with each pump chamber (21, 22) comprising a guide channel (81, 82) that is essentially annular and runs in the circumferential direction of the pump housing, said channel connecting inlets (31, 32) associated with pump chambers (21, 22) with outlets (41, 42) associated with the same pump chambers (21, 22), with each guide channel (81, 82) comprising a partial chamber (81a, 82a) which is offset relative to impeller (5) as viewed in the axial direction.

4. Centrifugal pump according to one of the foregoing claims with a stator (6) surrounding pump housing (2), said stator having electrical windings (61), with stator (6) further being designed as a bearing and drive stator, so that impeller (5) is drivable in the operating state by the magnetic interaction between stator (6) and rotor (53) and can also be mounted with zero contact in pump housing (2).

5. Centrifugal pump according to Claim 4 with rotor (53) being actively controllable or drivable in the operating state by means of electrical windings (61) of stator (6) with respect to three degrees of freedom and being mounted magnetically passively with respect to three additional degrees of freedom by reluctance forces in stator (6).

6. Centrifugal pump according to one of the foregoing claims with vanes (51, 52) each being angled, so that they each have a radial part (511) that extends outward in the radial direction in a straight line or in a curve, as well as an adjoining axial part (512) that extends essentially parallel to rotation axis (A) of impeller (5).

7. Centrifugal pump according to one of the foregoing claims with vanes (51) of one set having a different effective vane length than vanes (52) of the other set.

8. Centrifugal pump according to one of the foregoing claims with the two outlets (41, 42) being offset in the circumferential direction of pump housing (2).

9. Centrifugal pump according to one of the foregoing claims with axial height (H3, H4) of each guide channel (81, 82) increasing in the circumferential direction.

10. Centrifugal pump according to one of the foregoing claims with each inlet (31, 32) comprising an angled inlet channel (31a, 32a) which initially extends radially with respect to rotation axis (A) and then curves toward rotation axis (A) so that fluid can move from the axial direction to impeller (5).

11. Centrifugal pump according to one of the foregoing claims with rotor (53) having means (534, 535) for guiding the flow of the fluid.

12. Centrifugal pump according to one of the foregoing claims with means (536, 537) being provided to avoid fluid stagnation.

13. Centrifugal pump system with at least two centrifugal pumps (1, 1') each of which is designed according to one of the foregoing claims.

14. Centrifugal pump system according to Claim 13 with centrifugal pumps (1, 1') being connected with one another in such fashion that they form a hydraulic series circuit.

15. Blood pump especially an artificial heart with a centrifugal pump (1) according to one of Claims 1 to 12, or a centrifugal pump system (100) according to one of Claims 13-14.

## Revendications

1. Pompe centrifuge pour refouler un fluide, en particulier pour refouler du sang, comprenant un corps de pompe (2) qui comporte une première et une deuxième entrée (31, 32) ainsi qu'une première et une deuxième sortie (41, 42) du fluide, une roue à ailettes (5) disposée à l'intérieur du corps de pompe (2) et positionnée magnétiquement qui peut tourner autour d'un axe de rotation (A) et qui comprend un rotor (53) efficace magnétiquement en forme de disque ou d'anneau, ainsi que deux jeux d'ailettes (51, 52), un jeu d'ailettes (51, 52) étant respectivement prévu sur chacune des deux surfaces de délimitation axiales du rotor (53), **caractérisée en ce que** les deux sorties (41, 42) sont décalées l'une de l'autre dans le sens axial.

2. Pompe centrifuge selon la revendication 1, dans laquelle la roue à ailettes (5) délimite à l'intérieur du corps de pompe (2) deux chambres de pompe (21, 22) essentiellement séparées, et en ce que chaque chambre de pompe (21, 22) est associée respectivement à l'une des entrées (31, 32) et à l'une des sorties (41, 42).

3. Pompe centrifuge selon la revendication 2, dans laquelle chaque chambre de pompe (21, 22) comprend une conduite directrice (81, 82) essentiellement annulaire qui s'étend dans le sens circonférentiel du corps de pompe et qui relie l'entrée (31, 32) associée à la chambre de pompe (21, 22) à la sortie (41, 42) associée à la même chambre de pompe (21, 22), chaque conduite directrice (81, 82) comprenant un espace partiel (81a, 82a) qui, vu dans le sens axial, est décalé par rapport à la roue à ailettes (5).

4. Pompe centrifuge selon l'une des revendications précédentes comprenant un stator (6) qui entoure le corps de pompe (2) et comporte des enroulements (61) électriques, le stator (6) étant réalisé en tant que stator de positionnement et d'entraînement de sorte qu'en service, la roue à ailettes (5) peut être non seulement entraînée par l'interaction magnétique entre le stator (6) et le rotor (53), mais aussi être positionnée dans le corps de pompe (2) en l'absence de tout contact.

5. Pompe centrifuge selon la revendication 4, dans laquelle le rotor (53) peut être excité ou entraîné activement en service au moyen des enroulements (61) électriques du stator (6) par rapport à trois degrés de liberté et est positionné par rapport à trois autres degrés de liberté de manière magnétique et passive dans le stator (6) par des forces de réluctance.

6. Pompe centrifuge selon l'une des revendications précédentes, dans laquelle les ailettes (51, 52) sont réalisées respectivement de manière angulaire de sorte à présenter respectivement une partie radiale (511) qui s'étend en ligne droite ou en courbe dans le sens radial vers l'extérieur, ainsi qu'une partie axiale (512) qui lui est aboutée et qui s'étend pour l'essentiel parallèlement à l'axe de rotation (A) de la roue à ailettes (5).

7. Pompe centrifuge selon l'une des revendications précédentes, dans laquelle les ailettes (51) du premier jeu présentent une autre longueur effective que les ailettes (52) de l'autre jeu.

8. Pompe centrifuge selon l'une des revendications précédentes, dans laquelle les deux sorties (41, 42) sont décalées par rapport au sens circonférentiel du corps de pompe (2).

9. Pompe centrifuge selon l'une des revendications précédentes, dans laquelle la hauteur axiale (H3, H4) de chaque conduite directrice (81, 82) augmente dans le sens circonférentiel.

10. Pompe centrifuge selon l'une des revendications précédentes, dans laquelle chaque entrée (31, 32) comprend une conduite d'entrée (31a, 32a) angulaire qui s'étend tout d'abord essentiellement radialement par rapport à l'axe de rotation (A), puis se courbe dans le sens de l'axe de rotation (A) de sorte que le fluide peut parvenir dans la roue à ailettes (5) à partir du sens axial.

11. Pompe centrifuge selon l'une des revendications précédentes, dans laquelle le rotor (53) comprend des moyens (534 ; 535) d'écoulement du fluide.

12. Pompe centrifuge selon l'une des revendications précédentes, dans laquelle il est prévu des moyens (536 ; 537) pour éviter les stagnations de fluide.

13. Système des pompes centrifuges comprenant au moins deux pompes centrifuges (1, 1') dont chacune est réalisée selon l'une des revendications précédentes.

14. Système des pompes centrifuges selon la revendication 13, dans lequel les pompes centrifuges (1, 1') sont reliées l'une à l'autre de manière à former un montage en série hydraulique.

15. Pompe sanguine, en particulier coeur artificiel, comprenant une pompe centrifuge (1) selon l'une des revendications 1 à 12 ou un système des pompes centrifuges (100) selon l'une des revendications 13 à 14.
